# FASCICULE DE BREVET EUROPEEN

(11) **EP 2 374 482 B1**
(45) Date de publication et mention de la délivrance du brevet: **08.05.2019**
(21) Numéro de dépôt: 11160359.3
(22) Date de dépôt: 30.03.2011
(51) Int. Cl.: A61L 2/26, A61B 50/00

(54) **DISPOSITIF DE SCELLÉ DE BOÎTE DE STÉRILISATION**
VERSCHLUSS FÜR STERILISATIONSBEHÄLTER
LOCK FOR STERILISATION CONTAINER

(30) Priorité: 09.04.2010 FR 1052701
(43) Date de publication de la demande: 12.10.2011
(73) Titulaire: Sterlab, 06220 Vallauris (FR)
(72) Inventeur: Martel, Paul, 83700 St-Raphael (FR); Karacha, Philippe, 06330 Roquefort-Les-Pins (FR)
(74) Mandataire: Novagraaf Technologies

(56) Documents cités:
- EP-A2- 0 786 753
- US-A- 4 319 776
- US-A- 5 522 627
- US-A- 5 535 491
- US-A- 5 765 885
- US-A1- 2008 236 001

## Description

La présente invention a pour objet un dispositif de scellé de boîtes de stérilisation d'équipements, tels qu'en particulier des ustensiles médicaux et d'hôpitaux, effectué dans des appareils fermés telles que des autoclaves de type à pré-vide et utilisant la vapeur d'eau saturée sèche comme un gaz stérilisant, et dans lesquels on introduit des boîtes de stérilisation contenant lesdits équipements à stériliser.

Pour éviter que ces boîtes puissent être, par enlèvement de leurs couvercle, ouvertes après cette stérilisation, et tant qu'on n'a pas besoin des équipements qu'elles contiennent, il existe depuis longtemps des scellés qui apportent la preuve de leur non ouverture.

Pour cela, de tels scellés comme décrits entre autres dans les demandes de brevets et brevets US 5,522,627, EP 0 786 753 et US 4,319,779 comprennent en général une tige, apte à être glissée dans un dispositif de fermeture à oeillet tel que celui d'une boîte de stérilisation de la présente invention, et un bras pivotant sur une pièce support de l'extrémité proximale de la tige, et dont l'extrémité distale est apte à coopérer et se fixer sur celle distale de la tige d'une manière irréversible pour former avec cette tige et leur pièce support commune une boucle fermée empêchant l'ouverture du dispositif de fermeture.

Lesdits scellés sont majoritairement constitués de pièces en plastique, et l'emboîtement des extrémités de leur bras et de leur tige ne permet pas la réouverture de la boucle fermée ainsi formée, sauf en brisant une partie de cette pièce manuellement.

En général, deux scellés, dont un exemple est décrit ci-après, sont utilisés par boîte de stérilisation pour sécuriser les deux systèmes de fermeture situés sur deux côtés opposés du couvercle et du corps de la boîte.

D'autre part, chaque boîte de stérilisation est étiquetée avant ou après la stérilisation afin d'assurer son identification et la nature des équipements qu'elle contient, dans les stockages et/ou dans les zones d'utilisation.

Une étiquette, de surface assez importante, est placée pour cela sur la paroi de la boîte près d'un système de fermeture pour faciliter sa lecture quand les boîtes sont empilées, et est soit autocollante soit glissée dans un support.

L'inconvénient principal de ce mode d'identification est que l'étiquette soit doit être décollée de la boîte de stérilisation après usage et cette opération n'est non seulement pas pratique mais laisse également des traces de résidus de colle, voire d'étiquette sur la paroi de la boîte, soit peut sortir de son support et être enlevée facilement, pouvant alors créer un risque d'erreur.

Il est connu également, par ailleurs et certes dans d'autres domaines d'applications que les boîtes de stérilisation, des dispositifs de scellés, tels que dans le brevet US5535491, qui, dans le domaine par exemple des fixations de « skipass » sur un anorak, comporte une partie additionnelle ayant une surface apte à recevoir une étiquette d'identification lisible et pouvant être formé d'une languette plate rabattable par pliage le long d'une ligne formant charnière. On connaît également par le brevet US5765885 (ou son équivalent EP659149) des dispositifs de fermeture de sécurité comportant des doubles supports d'étiquettes rabattables l'un sur l'autre et dont l'un est détachable.

Le problème posé est d'éviter ainsi, en utilisant les connaissances de l'art antérieur, les inconvénients ci-dessus dans le domaine des boîtes de stérilisation et tout en préservant, lors de l'empilement de telles boîtes de stérilisation l'une sur l'autre cet inconvénient, tout en préservant, lors de l'empilement de telles boîtes de stérilisation l'une sur l'autre, la lecture des étiquettes d'identification de chaque boîte, et lors ou après l'ouverture de celle-ci la récupération de son étiquette.

Une solution au problème posé est un dispositif de scellé comprenant une tige apte à être glissée dans un dispositif de fermeture de boite de stérilisation et une pièce support de l'extrémité proximale de la tige, et tel que :
- ladite pièce support comprend une pièce de verrouillage apte à coopérer et se fixer sur l'extrémité distale de la tige d'une manière irréversible pour former avec celle-ci une boucle fermée empêchant l'ouverture du dispositif de fermeture et comporte une partie additionnelle à la partie support, nécessaire à la seule fonction de scellé, laquelle partie additionnelle comprend une surface de dimensions faciales de préférence supérieures à celles de la seule partie support, et suffisantes pour être apte à recevoir une étiquette d'identification lisible,
- ladite partie additionnelle est une languette plane rabattable sur la partie support de la tige qu'elle recouvre, par pliage le long d'une ligne formant charnière et offrant alors à la vue la surface opposée à celle faisant face à la partie support, et apte à recevoir l'étiquette d'identification,
- et suivant l'invention, cette partie additionnelle comporte une ouverture qui correspond, quand elle est rabattue, à l'endroit où se verrouille entre elle l'extrémité distale de la tige et la pièce de verrouillage.

De plus, la ligne de pliage peut être découpable, la partie additionnelle porte-étiquette étant apte ainsi à être séparée du dispositif de scellé.

Le résultat est un nouveau dispositif de scellé pour boîtes de stérilisation qui répond au problème posé et résout l'inconvénient principal des modes d'étiquetage actuels car l'étiquette soit n'a plus ainsi à être décollée, la surface de la boîte restant intacte, soit reste solidaire du scellé et donc de la boite sans risque d'être enlevée facilement.

Les figures 1 à 3 ci-jointes et la description ci-après représentent deux exemples de réalisation d'un tel dispositif de scellé suivant l'invention mais d'autres modes de réalisation sont cependant possibles dans le cadre de la portée de la présente invention.
La figure 1 est une vue d'ensemble d'un dispositif selon l'invention prêt à être scellé sur une boite de stérilisation ;
Le figure 2 est une vue d'un dispositif selon l'invention en position ouverte comme sur la figure 1 mais représenté sans boite de stérilisation ;
La figure 3 est une vue du dispositif de la figure 2 en position ouverte en ce qui concerne les parties aptes à effectuer le scellé mais avec sa partie additionnelle rabattue sur celles-ci.

Une boîte de stérilisation 10 comporte en général un corps de boîte 7 et un couvercle 6 amovible et apte à être maintenu sur le corps de boîte 7 par deux dispositifs de fermeture disposés chacun sur un côté opposé à l'autre de la forme en général parallélépipédique rectangle de la boîte.

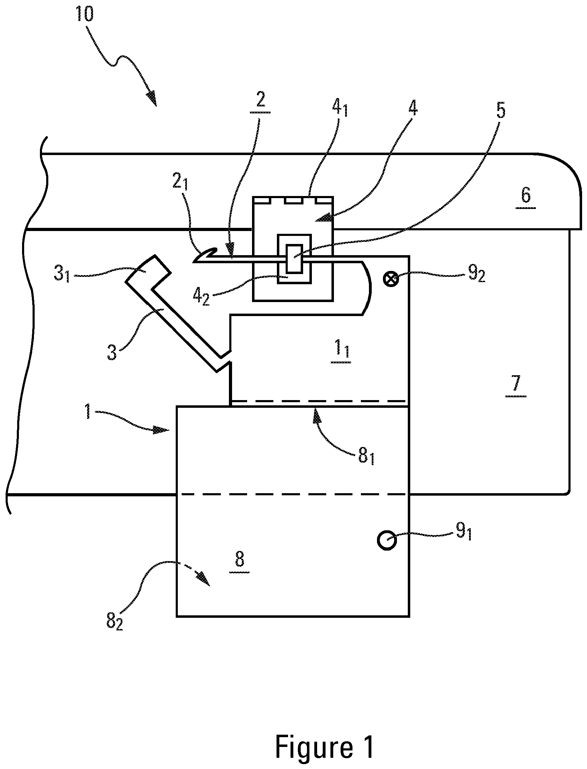

Un dispositif de fermeture, tel que représenté sur la figure 1, comporte en général un oeillet 5 apte à passer, en position de fermeture, dans une fenêtre 4₁ d'une languette articulée 4, ledit oeillet 5 et ladite languette 4 étant l'un ou l'une solidaire du couvercle 6 et l'autre du corps de boîte 7. Dans un autre mode de réalisation, on peut disposer d'un dispositif de fermeture composé de deux oeillets 5 chacun solidaire l'un du couvercle 6 et l'autre du corps de boîte 7 et leur l'orifice se faisant face en position de fermeture du couvercle 6 sur le corps de boîte 7.

La tige 2 du dispositif de scellé est apte à être ainsi passée dans le dispositif de fermeture, soit en l'occurrence au travers de l'orifice dudit oeillet 5 (ou des deux oeillets si le dispositif de fermeture en comporte deux, comme indiqué ci-dessus), et une pièce support 1₁ de l'extrémité proximale de la tige 2 comprend une pièce de verrouillage 3₁ qui est représentée ici comme l'extrémité distale d'un bras 3 pivotant sur la pièce support 1₁, et qui est apte à coopérer et se fixer sur l'extrémité distale 2₁ de la tige 2 d'une manière irréversible, tel que par emboîtement de celle-ci dans un réceptacle de verrouillage avec un système d'ergot empêchant le désemboîtement, l'ensemble formant une boucle fermée bloquant la languette 4 en position de fermeture.

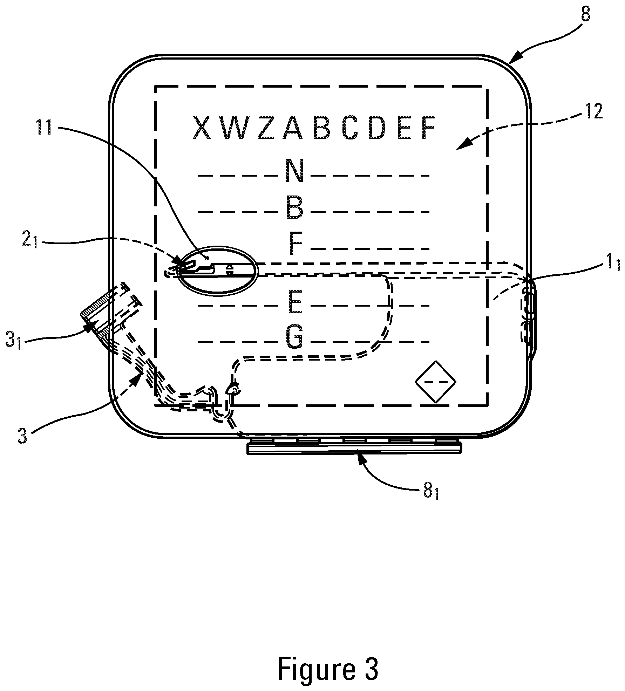

La pièce support 1 comporte une partie additionnelle 8 à celle 1₁ support nécessaire à la seule fonction de scellé : ladite partie support 1₁ comporte souvent une base présentant une surface sur laquelle pourrait être apposée une référence, mais elle est de petite dimension car sa raison d'être est d'une part de renforcer la base et/ou d'autre part d'assurer une meilleure saisie par l'opérateur qui manipule le dispositif de scellé souvent avec une seule main. Aussi une telle petite surface de la partie support 1₁, comme du reste représentée sur les figures jointes, ne permet pas d'apposer une étiquette lisible 12 d'identification complète : la qualification de lisible signifie qu'on doit pouvoir inscrire et lire facilement plusieurs références telles qu'un code, la date, les société intervenantes, et la nature ou même la liste des équipements que la boite de stérilisation contient, ce qui peut représenter jusqu'à environ 10 lignes d'inscription (comme schématisés par des lettres, dont la hauteur doit être de préférence au moins de l'ordre de 5 mm pour être justement facilement lisibles, et des tirets, sur la figure 3). Ladite partie additionnelle 8 de la présente invention comporte une surface 8₂ apte à recevoir une telle étiquette 12 d'identification qui fait en général de l'ordre de 5 cm de côté.

Cette partie additionnelle 8 est pour cela de dimensions faciales, soit en largeur et longueur telles que représentées dans le plan des figures, de préférence supérieures à celles de la seule partie support 1₁ et suffisantes pour recevoir l'étiquette d'identification 12 lisible : pour cela, sa largeur peut être plus importante que celle de la partie support 1₁ et sa longueur ou hauteur peut être telle qu'elle dépasse du fond du corps 7 de la boîte de stérilisation.

Aussi, pour permettre l'empilage des boîtes, cette partie additionnelle 8 porte étiquette est une languette plane en forme de plaque rabattable sur la partie support 1₁ de la tige 2, et dans le présent exemple de réalisation du bras 3 qu'elle recouvre, par pliage le long d'une ligne 8 formant charnière et offrant alors à la vue la surface 8₂ opposée à celle faisant face à la partie support 1₁, et apte à recevoir l'étiquette 12 d'identification. De plus, la partie additionnelle 8 ainsi rabattue en « portefeuille » protège la partie 1₁, formant scellé qu'elle recouvre.

Afin de pouvoir contrôler et vérifier, une fois la partie additionnelle 8 rabattue (et qui ne porte pas alors d'étiquette contrairement à ce qui est indiqué sur la figure 3 car celle-ci est souvent apposée à l'issue du cycle de stérilisation) que le scellé est bien verrouillé et l'est resté pendant toutes les opérations, cette partie additionnelle 8 comporte une ouverture 11 qui correspond, quand elle est rabattue, à l'endroit où se verrouillent entre elles l'extrémité distale 2₁ de la tige 2 et la pièce de verrouillage 3₁ qui peut être à l'extrémité du bras 3, comme représenté sur la figure 3, même si, sur cette figure, ces extrémités ne sont pas représentées verrouillées entre-elles : cette fenêtre 11 permet un contrôle visuel direct de la position de celles-ci.

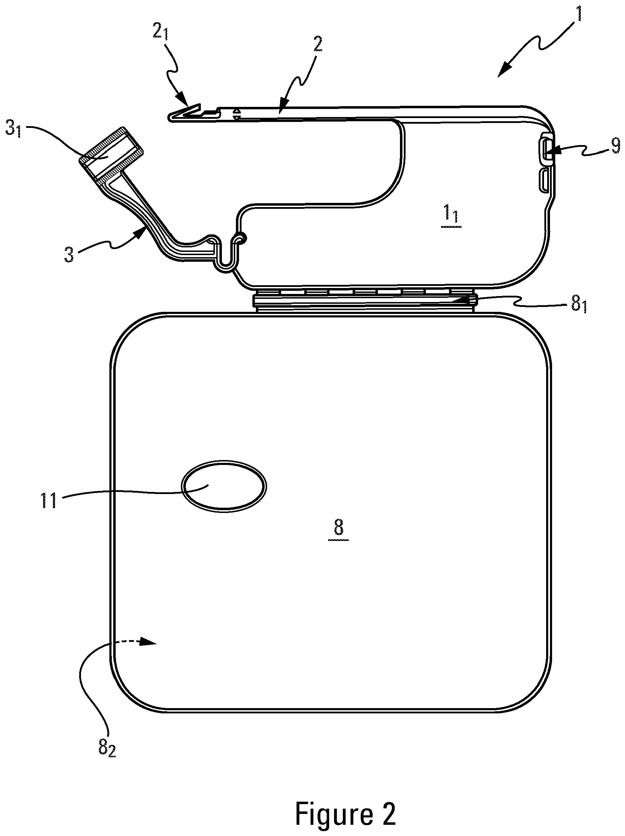

Pour assurer le maintien de cette partie additionnelle 8 rabattue contre la partie support 1₁, ces deux parties support 1₁ et additionnelle 8 comportent un dispositif de maintien 9 tel que par « clipsage » ou tout procédé similaire à un bouton pression, constitué d'éléments aptes à coopérer entre eux, soit comme sur la figure 1 avec deux éléments 9 de maintien chacun sur chaque partie support 1₁ et additionnelle 8, soit avec un seul élément 9, comme sur les figures 2 et 3, solidaire d'une des parties et en forme de rainure déformable dans laquelle vient se loger le bord de l'autre partie.

Suivant la figure 1, un des éléments solidaires de la partie support 1₁ peut être un ergot 9₂ faisant saillie à la surface de cette partie support 1₁ et l'autre élément 9₁ solidaire de la partie additionnelle rabattable peut être un simple orifice au travers duquel peut passer l'ergot 9₂ qui vient s'y bloquer.

De préférence, l'élément de maintien 9, ou de « clipsage » permet un maintien ou un « clipsage » réversible (soit sur la figure 1 par séparation de l'ergot 9₂ et de l'orifice 9₁, soit sur les figures 2 et 3 par déformation latérale de la rainure 9 pour libérer le bord de la partie additionnelle 8) : cet élément de maintien 9 est ainsi apte à permettre d'écarter la partie additionnelle 8 de la partie support 1₁ par rotation le long de la ligne 8₁.

De plus, la ligne 8₁ de pliage est découpable, la partie additionnelle 8 porte-étiquette étant apte ainsi à être séparée du dispositif de scellé pour conserver l'étiquette sans avoir à la décoller du dispositif de scellé.

## Revendications

1. Dispositif de scellé de boîtes de stérilisation comprenant une tige (2) apte à être glissée dans un dispositif de fermeture (5) d'une boîte de stérilisation, et une pièce support (1) de l'extrémité proximale de la tige (2)tel que :
- ladite pièce support (1) comprend une pièce de verrouillage (3₁) apte à coopérer et se fixer sur l'extrémité distale (2₁) de la tige (2) d'une manière irréversible pour former avec celle-ci une boucle fermée empêchant l'ouverture du dispositif de fermeture et comporte une partie additionnelle (8) à celle (1₁) support nécessaire à la seule fonction de scellé, laquelle partie additionnelle comprend une surface (8₂) de dimensions faciales suffisantes pour être apte à recevoir une étiquette d'identification (12) lisible,
- la partie additionnelle (8) est une languette plane rabattable sur la partie support (1₁) de la tige (2), qu'elle recouvre, par pliage le long d'une ligne (8₁) formant charnière et offrant alors à la vue la surface (8₂) opposée à celle faisant face à la partie support (1₁), et apte à recevoir l'étiquette d'identification,
**caractérisé en ce que** la partie additionnelle (8) comporte une ouverture (11) qui correspond, quand elle est rabattue, à l'endroit où se verrouillent entre-elles l'extrémité distale (2₁) de la tige (2) et la pièce de verrouillage (3₁).

2. Dispositif selon la revendication 1 **caractérisé en ce que** la surface (8₂) de la partie additionnelle (8) est de dimensions faciales supérieures à celles de la seule pièce support (1₁).

3. Dispositif selon l'une quelconque des revendications 1 ou 2, **caractérisé en ce que** les deux parties support (1₁) et additionnelle (8) comportent un dispositif de maintien (9) constitué d'éléments aptes à coopérer entre eux pour assurer le maintien de la partie additionnelle (8) rabattue contre la partie support (1₁).

4. Dispositif selon la revendication 3, **caractérisé en ce que** le dispositif de maintien (9) comporte deux éléments de maintien (9) chacun sur chaque partie support et additionnelle, séparables et aptes à permettre d'écarter la partie additionnelle (8) de la partie support (1₁) par rotation le long de la ligne (8₁).

5. Dispositif selon l'une quelconque des revendications 1 à 4, **caractérisé en ce que** la ligne (8₁) de pliage est découpable, la partie additionnelle (8) porte-étiquette étant apte à être ainsi séparée du dispositif de scellé.

## Patentansprüche

1. Verriegelungsvorrichtung von Sterilisationsbehältern, umfassend einen Schaft (2), der in der Lage ist, in eine Verschlussvorrichtung (5) eines Sterilisationsbehälters geschoben zu werden, und ein Trägerteil (1) des proximalen Endes des Schafts (2), sodass:
- das Trägerteil (1) ein Verriegelungsteil (3₁) umfasst, das in der Lage ist, mit dem distalen Ende (2₁) des Schafts (2) zusammenzuwirken und sich daran auf eine irreversible Weise zu befestigen, um damit einen geschlossenen Ring zu bilden, der das Öffnen der Verschlussvorrichtung verhindert und einen zum Trägerabschnitt (1₁), der für die einzige Versiegelungsfunktion notwendig ist, zusätzlichen Abschnitt (8) beinhaltet, wobei der zusätzliche Abschnitt eine Oberfläche (8₂) mit ausreichenden fazialen Abmessungen umfasst, um in der Lage zu sein, ein lesbares Identifikationsetikett (12) aufzunehmen,
- der zusätzliche Abschnitt (8) eine auf dem Trägerabschnitt (1₁) des Schafts (2) aufklappbare ebene Lasche ist, den sie durch Falten entlang einer scharnierbildenden und daher die dem Trägerabschnitt (1₁) zugewandte gegenüberliegende Oberfläche (8₂) zur Ansicht bietenden Linie (8₁) abdeckt, und die in der Lage ist, das Identifikationsetikett aufzunehmen,
**dadurch gekennzeichnet, dass** der zusätzliche Abschnitt (8) eine Öffnung (11) umfasst die, wenn er aufgeklappt wird, der Stelle entspricht, an der sich das distale Ende (2₁) des Schafts (2) und das Verriegelungsteil (3₁) miteinander verriegeln.

2. Vorrichtung nach Anspruch 1, **dadurch gekennzeichnet, dass** die Oberfläche (8₂) des zusätzlichen Abschnitts (8) faziale Abmessungen aufweist, die größer sind als die des einzigen Trägerteils (1₁).

3. Vorrichtung nach einem der Ansprüche 1 oder 2, **dadurch gekennzeichnet, dass** sowohl der Trägerabschnitt (1₁) als auch der zusätzliche Abschnitt (8) eine Haltevorrichtung (9) umfassen, die aus Elementen besteht, die in der Lage sind, untereinander zusammenzuwirken, um das Halten des zusätzlichen, gegen den Trägerabschnitt (1₁) aufgeklappten, Abschnitts (8) zu gewährleisten.

4. Vorrichtung nach Anspruch 3, **dadurch gekennzeichnet, dass** die Haltevorrichtung (9) zwei Halteelemente (9) umfasst, jedes auf jedem des Trägerabschnitts und des zusätzlichen Abschnitts, trennbar und in der Lage, zu erlauben, den zusätzlichen Abschnitt (8) des Trägerabschnitts (1₁) durch Rotation entlang der Linie (8₁) fernzuhalten.

5. Vorrichtung nach einem der Ansprüche 1 bis 4, **dadurch gekennzeichnet, dass** die Faltungslinie (8₁) auskoppelbar ist, wobei somit der etiketttragende zusätzliche Abschnitt (8) in der Lage ist, von der Versiegelungsvorrichtung getrennt zu werden.

## Claims

1. Device for locking sterilisation boxes comprising a rod (2) able to be slid into a device for closing (5) a sterilisation box, and a support part (1) of the proximal end of the rod (2) such that:
- said support part (1) comprises a locking part (3₁) able to engage and be fastened irreversibly on the distal end (2₁) of the rod (2) in order to form with the latter a closed loop that prevents the opening of the device for closing and comprises a portion that is additional (8) to that (1₁) for the support required solely for the sealing function, said additional portion comprises a surface (8₂) with facial dimensions that are sufficient to be able to receive a legible identification label (12),
- the additional portion (8) is a flat tab that can be folded over the support portion (1₁) of the rod (2), that it covers, by folding along a line (8₁) that forms a hinge and which then offers as seen the surface (8₂) opposite that facing the support portion (1₁), and able to receive the identification label,
**characterised in that** the additional portion (8) comprises an opening (11) that corresponds, when it is folded back, to the location where the distal end (2₁) of the rod (2) and the locking part (3₁) are locked together.

2. Device according to claim 1 **characterised in that** the surface (8₂) of the additional portion (8) is of facial dimensions greater than those of the support part (1₁) alone.

3. Device according to any of claims 1 or 2, **characterised in that** the two support (1₁) and additional (8) portions comprise a device for maintaining (9) comprised of elements that can engage with one another in order to provide the maintaining of the additional portion (8) folded back against the support portion (1₁) .

4. Device according to claim 3, **characterised in that** the device for maintaining (9) comprises two maintaining elements (9) each one on each support and additional portion, able to be separated and able to make it possible to separate the additional portion (8) from the support portion (1₁) via rotation along the line (8₁).

5. Device according to any of claims 1 to 4, **characterised in that** the folding line (8₁) can be cut, the additional label-holding portion (8) thus being able to be separated from the sealing device.
